# EUROPEAN PATENT APPLICATION

(11) **EP 3 005 931 A1**
(43) Date of publication of application: **13.04.2016**
(21) Application number: 14804701.2
(22) Date of filing: 15.05.2014
(51) Int. Cl.: A61B 1/00

(54) **CALIBRATION ASSISTING DEVICE, BENDING SYSTEM AND CALIBRATION METHOD**

(30) Priority: 29.05.2013 JP 2013113205
(71) Applicant: Olympus Corporation, Shibuya-ku Tokyo 151-0072 (JP)
(72) Inventor: SATO, Ken, Shibuya-ku Tokyo 151-0072 (JP)
(74) Representative: Winter, Brandl, Fürniss, Hübner, Röss, Kaiser, Polte - Partnerschaft mbB
(86) International application number: PCT/JP2014/062957
(87) International publication number: WO 2014/192553

(57) **Abstract**

A calibration assist apparatus is an apparatus for use in a curving system (1), the curving system including an elongated flexible portion (21) and a curve detection unit (22) which is provided in the flexible portion and which is configured to detect a curving amount of the flexible portion. The calibration assist apparatus assists in calibrating the curve detection unit. The calibration assist apparatus includes a calibrator (30) configured to restrain a deformation and a movement of the flexible portion in both directions along a first axis perpendicular to a longitudinal axis of the flexible portion and restrain a deformation and a movement of the flexible portion in at least one direction along a second axis perpendicular to the longitudinal axis and the first axis.

## Description

### Technical Field

The present invention relates to a calibration assist apparatus which assists in calibrating a detector for detecting a curving amount of an elongated flexible portion, a curving system including this calibration assist apparatus, and a calibration method.

### Background Art

For example, in a curving system having an elongated flexible portion such as an insertion tube of an endoscope, this flexible portion may be provided with a curve detection unit configured to detect a curving amount to estimate a curving shape of the flexible portion. For such a curve detection unit to accurately operate, the curve detection unit needs to be calibrated.

For example, Jpn. Pat. Appln. KOKAI Publication No. 2003-070718 discloses an electronic endoscope in which, for example, about 5 to 30 curve detection units are provided over the entire length of a flexible insertion tube at intervals of, for example, about several centimeters in the axis line direction of the flexible insertion tube. Jpn. Pat. Appln. KOKAI Publication No. 2003-070718 discloses that the curve detection units are calibrated by winding the flexible insertion tube along the outer circumference of a circular cylindrical drum having a known radius R.

Accurately calibrating the curve detection unit is important to precisely detect a curving amount of the flexible portion. For example, as disclosed in Jpn. Pat. Appln. KOKAI Publication No. 2003-070718, when the drum is used to make a calibration, the flexible portion runs along the outer circumference of the drum, and is not restrained, for example, on the circumferential surface of the drum. That is, the flexible portion can meander on the circumferential surface of the drum. If the flexible portion, for example, meanders at the time of a calibration, an accurate calibration may not be made.

### Summary of Invention

The present invention is intended to provide a calibration assist apparatus, a curving system, and a calibration method to make an accurate calibration.

To achieve the above described object, according to an aspect of the invention, a calibration assist apparatus is an apparatus for use in a curving system, the curving system including an elongated flexible portion and a curve detection unit which is provided in the flexible portion and which is configured to detect a curving amount of the flexible portion, the calibration assist apparatus assisting in calibrating the curve detection unit. The calibration assist apparatus includes a calibrator configured to restrain a deformation and a movement of the flexible portion in both directions along a first axis perpendicular to a longitudinal axis of the flexible portion and restrain a deformation and a movement of the flexible portion in at least one direction along a second axis perpendicular to the longitudinal axis and the first axis.

To achieve the above described object, according to an aspect of the invention, a curving system includes an elongated flexible portion; a curve detection unit which is provided in the flexible portion and which detects a curving amount of the flexible portion; a calibrator configured to restrain a deformation and a movement of the flexible portion in both directions along a first axis perpendicular to a longitudinal axis of the flexible portion and restrain a deformation and a movement of the flexible portion in at least one direction along a second axis perpendicular to the longitudinal axis and the first axis; and a calibration operation unit which calibrates the curve detection unit based on an output of the curve detection unit in a situation in which the flexible portion is restrained by the calibrator and a shape of the flexible portion in the situation in which the flexible portion is restrained.

To achieve the above described object, according to an aspect of the invention, a calibration method includes restraining a deformation and a movement of an elongated flexible portion in both directions along a first axis perpendicular to a longitudinal axis of the flexible portion and restrain a deformation and a movement of the flexible portion in at least one direction along a second axis perpendicular to the longitudinal axis and the first axis using a calibrator; acquiring an output of a curve detection unit which is provided in the flexible portion and which is configured to detect a curving amount of the flexible portion in a situation in which the flexible portion is restrained by the calibrator; and calibrating the curve detection unit based on a shape of the flexible portion in the restraint state and the output.

According to the present invention, a flexible portion is restrained, and it is therefore possible to provide a calibration assist apparatus, a curving system, and a calibration method which can make an accurate calibration.

### Brief Description of Drawings

FIG. 1 is a block diagram showing a configuration example of an endoscope system according to a first embodiment;
FIG. 2A is a diagram illustrating a fiber sensor;
FIG. 2B is a diagram illustrating the fiber sensor;
FIG. 2C is a diagram illustrating the fiber sensor;
FIG. 3 is a diagram showing an overview of a configuration example of an insertion portion;
FIG. 4A is a diagram showing an overview of a configuration example of a first calibrator according to the first embodiment;
FIG. 4B is a diagram showing an overview of a configuration example of a second calibrator according to the first embodiment;
FIG. 4C is a diagram showing an overview of a configuration example of a third calibrator according to the first embodiment;
FIG. 5 is a flowchart showing an example of processing regarding a calibration operation;
FIG. 6 is a schematic diagram showing an example of the relation between the insertion portion and the calibrator when calibration is made;
FIG. 7 is a diagram showing an example of indices for adjusting the positional relation between the insertion portion and the calibrator;
FIG. 8 is a diagram showing an overview of a configuration example of a calibrator according to a first modification of the first embodiment;
FIG. 9 is a diagram showing an overview of a configuration example of a calibrator according to a second modification of the first embodiment;
FIG. 10 is a diagram showing an overview of a configuration example of a calibrator according to a second embodiment;
FIG. 11 is a diagram showing an overview of a configuration example of a calibrator according to a modification of the second embodiment;
FIG. 12 is a block diagram showing a configuration example of an endoscope system according to a third embodiment; and
FIG. 13 is a block diagram showing a configuration example of an endoscope system according to a modification of the third embodiment.

### Description of Embodiments

### [First Embodiment]

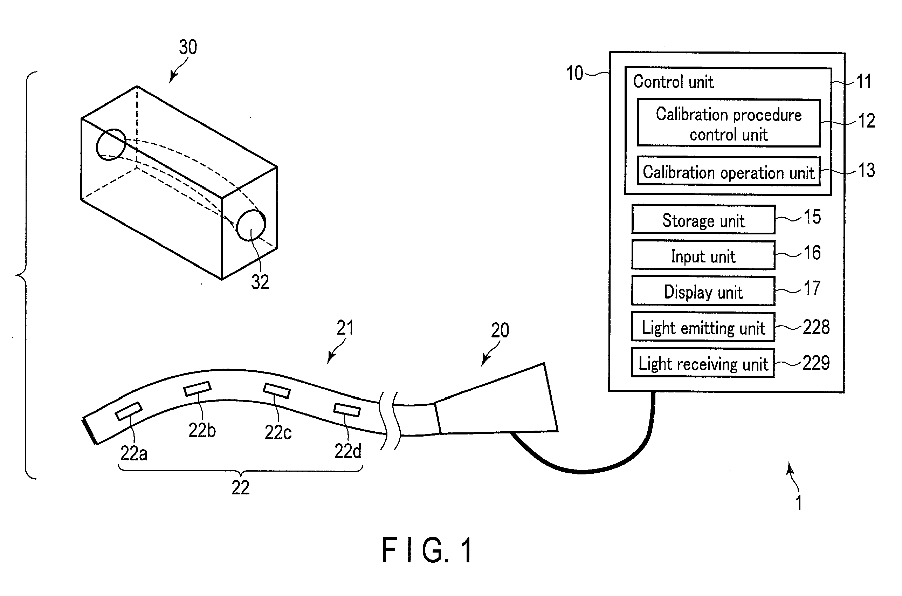

A first embodiment is described with reference to the drawings. FIG. 1 shows an overview of a configuration example of an endoscope system 1 as a curving system according to the first embodiment. The endoscope system 1 comprises a main unit 10, an endoscope 20, and a calibrator 30.

The endoscope 20 is configured to observe, for example, a body cavity. The endoscope 20 comprises an elongated flexible insertion portion 21. An unshown illumination window for emitting illumination light and an unshown camera or the like including an image pickup device for imaging a subject are provided at the distal end of the insertion portion 21. The insertion portion 21 is inserted into, for example, the body cavity, images the inside of the body cavity, and sends image data to the main unit 10. An image based on this image data is displayed on a later-described display unit 17 of the main unit 10.

A curve detection unit 22 for detecting a curving amount of the insertion portion 21 is provided in the insertion portion 21. The curve detection unit 22 is a sensor group including at least one curve angle sensor disposed in the insertion portion 21. As shown in FIG. 1, the curve detection unit 22 includes, for example, a first detector 22a, a second detector 22b, a third detector 22c, and a fourth detector 22d. The position where each detector is provided in the insertion portion 21, for example, the distance from the distal end of the insertion portion 21 is known. Although four detectors are schematically shown in FIG. 1, any number of detectors may be provided.

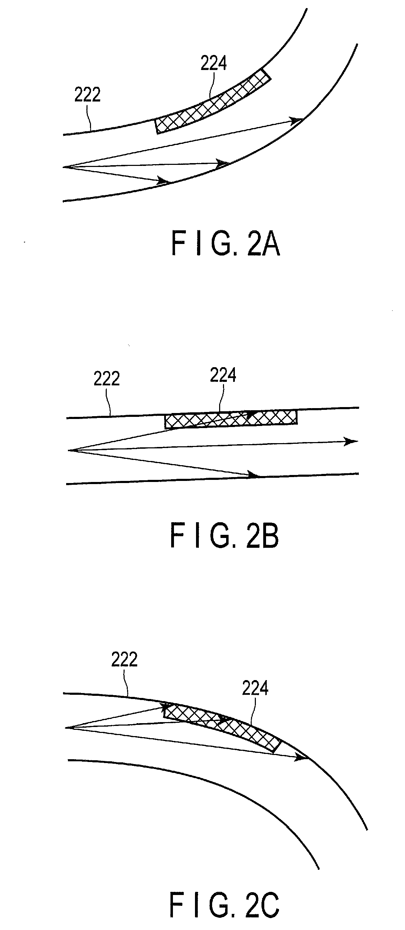

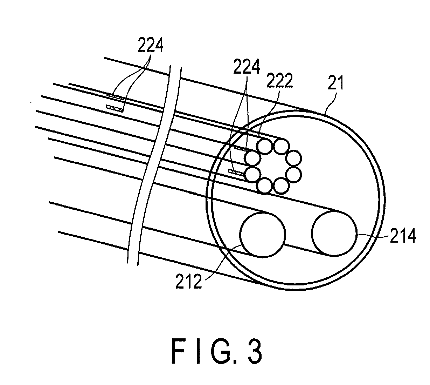

For example, fiber sensors can be used as the detectors included in the curve detection unit 22. An example of the fiber sensor is described with reference to FIG. 2A, FIG. 2B, FIG. 2C, and FIG. 3. An optical fiber 222, a light emitting unit 228 which emits light guided by the optical fiber 222, and a light receiving unit 229 which receives light guided by the optical fiber are used in the fiber sensor. The light emitting unit 228 and the light receiving unit 229 are provided in, for example, the main unit 10.

The operation principle of the fiber sensor is described. A detection region 224 is provided in the curve detection unit 22. In the detection region 224, the cladding of the optical fiber 222 is removed so that a core is exposed, and this part is coated with a light absorbing member. As a result, the amount of light guided by the optical fiber 222 changes depending on the state of the curving of the optical fiber 222.

For example, when the optical fiber 222 is curved so that the detection region 224 comes inside as shown in FIG. 2A, the light transmission rate by the optical fiber 222 is higher. In contrast, when the optical fiber 222 is curved so that the detection region 224 comes outside as shown in FIG. 2C, the light transmission rate by the optical fiber 222 is lower. When the optical fiber 222 is not curved as shown in FIG. 2B, the light transmission rate by the optical fiber 222 is lower than that in the case shown in FIG. 2A and higher than that in the case shown in FIG. 2C. Such an optical fiber 222 is inserted through the insertion portion 21. Light emitted from the light emitting unit 228 provided in the main unit 10 enters the optical fiber 222, passes through the detection region 224, and is then again guided to the main unit 10, and is detected by the light receiving unit 229. The light receiving unit 229 measures the amount of the light guided by the optical fiber 222. A curving amount of the insertion portion 21 in the region where the detection region 224 is provided is calculated on the basis of the measured amount of the received light.

As shown in FIG. 3, a bundle of optical fibers 222 are disposed in the insertion portion 21. To detect curving amounts in two directions (e.g., an X-axis direction and a Y-axis direction) that intersect at orthogonal angles in each part of the insertion portion 21, the optical fiber 222 in which the detection region 224 is provided in one direction (e.g., the X-axis direction) and the optical fiber 222 in which the detection region 224 is provided in a direction (e.g., the Y-axis direction) that intersects at orthogonal angles with the one direction are provided in pairs in the insertion portion 21. Moreover, the optical fibers 222 in which the detection regions 224 are provided at different positions in the longitudinal axis direction of the insertion portion 21 to correspond to, for example, the first detector 22a, the second detector 22b, the third detector 22c, and the fourth detector 22d are provided in the insertion portion 21. In FIG. 3, an illumination light optical fiber 212 which transmits the illumination light emitted from the distal end of the insertion portion 21, and a wiring line 214 for the image pickup device provided at the distal end of the insertion portion 21 are drawn in addition to the optical fibers 222.

The endoscope system 1 according to the present embodiment is designed to facilitate calibration that maintains high precision in the detection of the curving amount of the insertion portion 21 by the curve detection unit 22 described above. The configuration and operation for such calibration are described.

The calibrator 30 included in a calibration assist apparatus according to the present embodiment is described. As shown in FIG. 1, a through-hole 32 is provided in the calibrator 30. The inside diameter of this through-hole 32 is slightly larger than the outside diameter of the insertion portion 21. This through-hole 32 is curved in the shape of a circular arc having a known curvature radius or is straight. The insertion portion 21 is inserted into the through-hole 32. When the insertion portion 21 is inserted in the through-hole 32, the insertion portion 21 is restrained in a curving shape with the known curvature radius or in a straight state. That is, the insertion portion 21 can move in the longitudinal axis direction of the insertion portion 21 and rotate around the longitudinal axis, but other movements of the insertion portion 21 are restricted. This state is referred to as a restraint state. The curve detection unit 22 is calibrated while the insertion portion 21 is in the restraint state. The length of the through-hole 32 is shorter than that of the insertion portion 21, and is longer than the distance between the detectors, for example, the distance between the first detector 22a and the second detector 22b. That is, the calibration is made for each part of the insertion portion 21. The relatively short length of the calibrator 30 is advantageous to easy handling of the calibrator 30. For example, a user can easily sterilize the calibrator 30. The shape of the through-hole 32 of the calibrator 30 is important, and the calibrator 30 may have any outer shape and is not limited to the rectangular parallelepiped shown in the drawing.

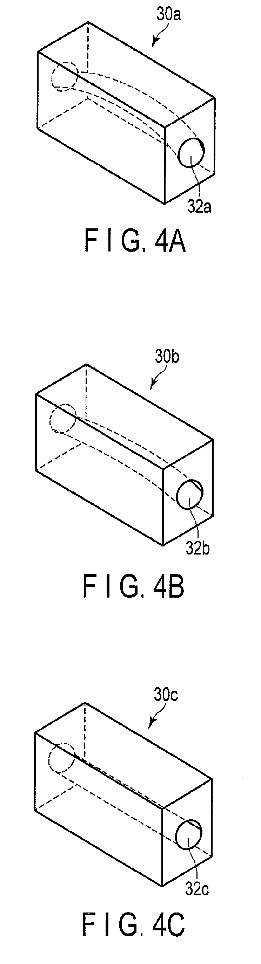

FIG. 4A, FIG. 4B, and FIG. 4C are diagrams showing examples of overviews of various calibrators 30. FIG. 4A shows an overview of a first calibrator 30a. The first calibrator 30a has a first through-hole 32a. The curvature radius of the first through-hole 32a is Ra. FIG. 4B shows an overview of a second calibrator 30b. The second calibrator 30b has a second through-hole 32b. The curvature radius of the second through-hole 32b is Rb. Here, there is a relation Ra<Rb. FIG. 4C shows an overview of a third calibrator 30c. The third calibrator 30c has a third through-hole 32c. The third through-hole 32c is straight. That is, a curvature radius Rc of the third through-hole 32c is infinite.

Indices indicating a curvature and a curving direction are indicated in each of the calibrators 30. That is, the curvatures and curving directions of, for example, the first through-hole 32a, the second through-hole 32b, and the third through-hole 32c are indicated in the first calibrator 30a, the second calibrator 30b, and the third calibrator 30c by, for example, characters, symbols, or colors. The user can easily recognize the different calibrators 30 by the indices.

In other words, the shapes of these calibrators 30 are expressed as follows: When the insertion portion 21 is inserted into the through-hole 32, the longitudinal axis of the through-hole 32 corresponds to the longitudinal axis of the insertion portion 21. At a given position of the through-hole 32, a given first axis perpendicular to the longitudinal axis of the through-hole 32 is defined. The through-hole 32 restrains the deformation and movement of the inserted insertion portion 21 in both directions (e.g., both right and left directions) along the first axis. Moreover, a second axis perpendicular to both the longitudinal axis of the through-hole 32 and the first axis is defined. The through-hole 32 restrains the deformation and movement of the inserted insertion portion 21 in at least one direction (e.g., upward or downward direction) along the second axis.

In addition to the light emitting unit 228 and the light receiving unit 229 that have been mentioned above, the main unit 10 comprises a control unit 11, a storage unit 15, an input unit 16, and the display unit 17. The control unit 11 controls various parts and performs calculations regarding a calibration operation according to the present embodiment. A calibration procedure control unit 12 and a calibration operation unit 13 are provided in the control unit 11. The calibration procedure control unit 12 operates so that the calibration operation is performed in accordance with a predetermined procedure. The calibration procedure control unit 12 controls the procedure of the calibration operation, for example, to display instructions to the user regarding the calibration on the later-described display unit 17 or to output information regarding the calibration to the calibration operation unit 13. The calibration operation unit 13 calculates calibration data regarding the relation between the shape of the insertion portion 21 and the amount of received light on the basis of the curvature of the through-hole 32 and the state of the insertion portion 21 including, for example, an insertion amount and a rotation amount acquired from the calibration procedure control unit 12, and the amount of received light acquired from the light receiving unit 229.

The storage unit 15 stores programs and parameters regarding various operations according to the present embodiment. The storage unit 15 also stores the calibration data calculated by the calibration operation unit 13. When the endoscope system 1 is used, the calibration data stored in the storage unit 15 is read, and the curving amount of the insertion portion 21 is calibrated. The input unit 16 acquires an instruction from the user. The input unit 16 includes a calibration start button which indicates that, for example, the user may fix the positional relation between the insertion portion 21 and the calibrator 30 to a predetermined relation and acquire data for calibration. As the input unit 16, a keyboard, a touch panel, and a mouse, for example, can be used in addition to a button switch. The display unit 17 includes, for example, a liquid crystal display to display various images.

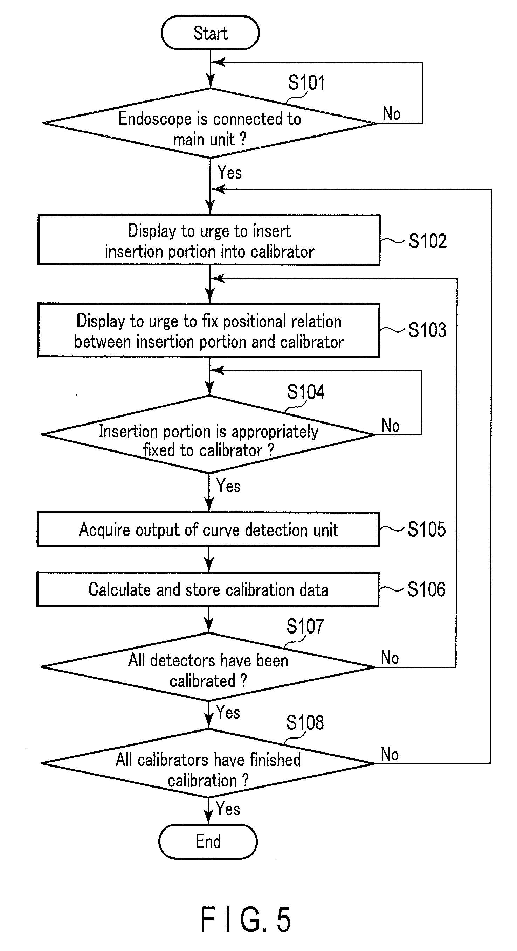

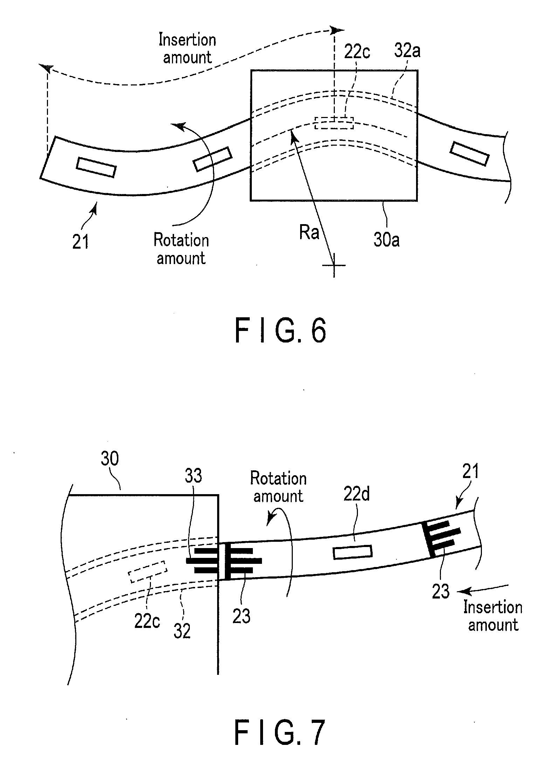

Next, the calibration operation in the endoscope system 1 according to the present embodiment is described with reference to FIG. 5 and FIG. 6.

In the case shown in the present embodiment, all the detectors included in the curve detection unit 22 are calibrated by all the prepared calibrators 30, for example, the first calibrator 30a, the second calibrator 30b, and the third calibrator 30c. For example, the insertion portion 21 is inserted into the first calibrator 30a from the distal side of the insertion portion 21, that is, from the first detector 22a in order, and calibration is then made in order.

Processing performed in the main unit 10 in the present embodiment is described with reference to a flowchart shown in FIG. 5. In step S101, the control unit 11 determines whether the endoscope 20 is connected to the main unit 10. When it is determined that the endoscope 20 is not connected to the main unit 10, the processing repeats step S101 and waits. In contrast, when it is determined that the endoscope 20 is connected to the main unit 10, the processing progresses to step S102.

In step S102, the control unit 11 displays, on the display unit 17, characters or graphics to urge the user to insert the insertion portion 21 into the predetermined calibrator 30. For example, a message "Insert insertion tube into first calibrator" is displayed on the display unit 17. Looking at this indication, the user inserts the insertion portion 21 into the calibrator 30 displayed on the display unit 17.

In step S103, the control unit 11 displays, on the display unit 17, characters or graphics to urge the user to set the positional relation between the insertion portion 21 and the calibrator 30 to a predetermined positional relation. FIG. 6 is a diagram showing the relation between the insertion portion 21 and the calibrator 30 when the calibration is made, and illustrates a case in which the first calibrator 30a is used to calibrate the third detector 22c. Here, the positional relation between the insertion portion 21 and the calibrator 30 is determined by an insertion amount and a rotation amount as shown in FIG. 6. The insertion amount is an amount indicating the position of the insertion portion 21 in the longitudinal direction of the insertion portion 21, that is, in the direction in which the insertion portion 21 is inserted into the through-hole 32. The insertion amount can be defined by, for example, the length from the distal end of the insertion portion 21 to the center of the through-hole 32 along the longitudinal axis of the insertion portion 21. The rotation amount is an amount indicating an angle of rotation around the longitudinal axis of the insertion portion 21.

In step S103, the control unit 11 displays, on, for example, the display unit 17, indications such as "insert xx cm" or "align the index of the first detector with the index of the calibrator". It should be understood that not only character indications but also graphics, for example, may be used. Which of the detectors in the curve detection unit 22 is making the calibration may be shown.

For example, indices shown in FIG. 7 are added to the calibrator 30 and the insertion portion 21. The user aligns an index 33 indicated on the calibrator 30 with an index 23 indicated on the insertion portion 21, resulting that the position of the insertion portion 21 in the longitudinal direction and the rotation amount in the circumferential direction have a predetermined relation with the calibrator 30. The user uses the index 33 of the calibrator and the index 23 of the insertion portion 21 to set the positional relation between the insertion portion 21 and the calibrator 30 to a predetermined positional relation. According to the index 33 of the calibrator and the index 23 of the insertion portion 21, alignment is easier for the user. The indices shown in FIG. 7 are illustrative only, and indices may have any shape, design, or color as long as such indices function in a similar manner. Thus, for example, the indices 23 and 33 function as position identifying members to identify the position of the curve detection unit 22 relative to the calibrator 30, or rotation identifying members to identify the rotation amount of the curve detection unit 22 relative to the calibrator 30.

For example, the user presses the calibration start button included in the input unit 16 when the insertion portion 21 is appropriately fixed to the calibrator 30 as displayed on the display unit 17. In step S104, the control unit 11 determines whether the insertion portion 21 is appropriately fixed to the calibrator 30. That is, the control unit 11 determines whether the calibration start button has been pressed by the user. When it is determined that the insertion portion 21 is not appropriately fixed to the calibrator 30, the processing repeats step S104 and waits. In contrast, when it is determined that the insertion portion 21 is fixed to the calibrator 30, the processing progresses to step S105. That is, when the region of the insertion portion 21 including the detector to be calibrated at present is fixed to have a curve with a known curvature radius, the processing progresses to step S105.

In step S105, the control unit 11 acquires the output of the detector which is being calibrated in the curve detection unit 22. In step S106, the control unit 11 calculates calibration data regarding the calibration of the curve detection unit 22; for example, the relation between the output of the detector and the curvature radius of the calibrator 30. The control unit 11 stores the calibration data in the storage unit 15.

In step S107, the control unit 11 determines whether all the detectors have been calibrated. That is, the control unit 11 determines whether all the detectors have been calibrated; for example, the first detector 22a, the second detector 22b, the third detector 22c, and the fourth detector 22d in order. When it is determined that all the detectors have not been calibrated, the processing returns to step S103. In step S103, such characters etc. are displayed on the display unit 17 as to fix the insertion portion 21 to the calibrator 30 so that the next detector will be restrained by the calibrator 30. Processing similar to that described above is then performed. When it is determined in step S107 that all the detectors have been calibrated, the processing progresses to step S108.

In step S108, the control unit 11 determines whether calibration that uses all the detectors have been finished. That is, the control unit 11 determines whether all the calibrators have been used; for example, the first calibrator 30a, the second calibrator 30b, and the third calibrator 30c in order. When it is determined that the calibration that uses all the detectors have not been finished, the processing returns to step S102. In step S102, characters, for example, are displayed on the display unit 17 to urge the user to insert the insertion portion 21 into the next calibrator 30. When it is determined in step S108 that all the calibrators have been used, the processing is finished.

According to the present embodiment, each of the detectors included in the curve detection unit 22 is fixed in a known curving state by the through-hole 32 of each calibrator 30. The outside diameter of the insertion portion 21 is substantially equal to the inside diameter of this through-hole 32, and the insertion portion 21 is therefore surely restrained. The output of the curve detection unit 22 in the known curving state is thus acquired, so that the curve detection unit 22 is precisely calibrated. Such a calibration operation may be performed, for example, at the time of shipment, or may be performed by the user before use.

At least part of the calibrator 30 may be transparent so that the state of the inserted insertion portion 21 can be recognized, and the inside of the through-hole 32 may be visible from the outside of the calibrator 30. A cutout which functions as a window may also be provided in part of the calibrator 30. If the user can visually recognize the insertion portion 21 inside the calibrator 30, it is easier for the user to fix the insertion portion 21 to the calibrator 30.

Although calibration is made while the insertion portion 21 is sequentially inserted into the through-hole 32 of the calibrator 30 in the above example, calibration may be made while the insertion portion 21 is sequentially pulled out of the calibrator 30. In this instance, calibration is made from the proximal side of the insertion portion 21 in order, that is, from the fourth detector 22d in order. Calibration may be made while the insertion portion 21 is both inserted into and pulled out of the calibrator 30. For example, if there is a change of the rotation amount between the insertion and pulling of the insertion portion 21 in and out of the calibrator 30, calibration data are acquired in different curving states, so that calibration with a higher degree of precision can be made.

Although the calibration of the curve detection unit 22 provided in the insertion portion 21 of the endoscope 20 has been described here, the technique according to the present embodiment is not only applicable to endoscopes but also applicable to curve detection units provided in various elongated flexible portions. The technique according to the present embodiment is applicable to, for example, catheters and treatment instruments, and is not only applicable to medical equipment but also applicable to various manipulators.

### [First Modification of First Embodiment]

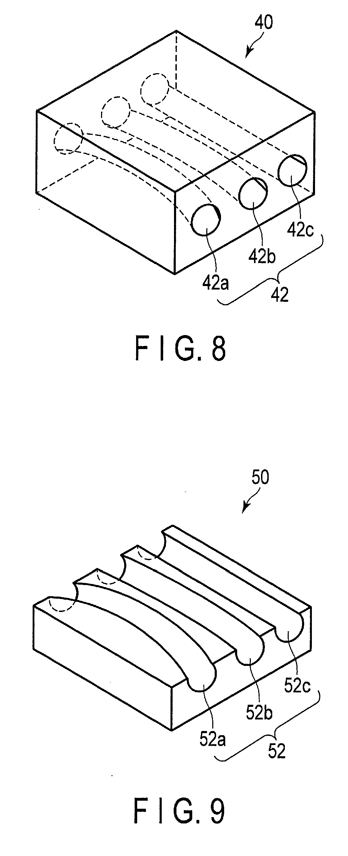

A first modification of the first embodiment is described. The differences between the first modification and the first embodiment are described here, and the same parts are provided with the same reference signs and are not described. As shown in FIG. 8, a calibrator 40 according to the present modification is provided with through-holes 42 having different curvature radii in one calibrator 40. That is, the calibrator 40 is provided with a first through-hole 42a, a second through-hole 42b, and a third through-hole 42c. The curvature radius of the first through-hole 42a is smaller than the curvature radius of the second through-hole 42b. The curvature radius of the second through-hole 42b is smaller than the curvature radius of the third through-hole 42c. The inside diameter of this through-hole 42 is slightly larger than the outside diameter of the insertion portion 21. That is, one calibrator 40 according to the present modification takes charge of the functions of the first calibrator 30a, the second calibrator 30b, and the third calibrator 30c according to the first embodiment. The configuration is similar in other respects to that in the first embodiment. The calibrator 40 according to the present modification functions in a similar manner to the calibrator 30 according to the first embodiment. Thus, the first through-hole 42a, the second through-hole 42b, and the third through-hole 42c function as restraining portions configured to restrain the insertion portion 21.

While three calibrators 30 are provided in the first embodiment, one calibrator 40 is provided in the present modification. Thus, according to the present modification, the storage and management of the calibrator 40 are easier. In other respects, advantageous effects similar to those in the first embodiment are also obtained according to the present modification.

Although the calibrator 40 is provided with multiple through-holes 42 in the case described in the above example, any calibrator is possible as long as one calibrator has through-holes having multiple known curvature radii. For example, a calibrator may be configured so that a dial is turned to change the curvature radius of the through-hole in response to the dial.

### [Second Modification of First Embodiment]

A second modification of the first embodiment is described. The differences between the second modification and the first embodiment are described here, and the same parts are provided with the same reference signs and are not described. In the first modification of the first embodiment, the first through-hole 42a or the like restrains the shape of the insertion portion 21 in the calibrator 40. In contrast, as shown in FIG. 9, a calibrator 50 according to the present modification is provided with a groove 52 having, for example, a semicircular sectional shape to restrain the shape of the insertion portion 21. That is, the calibrator 50 is provided with a first groove 52a, a second groove 52b, and a third groove 52c. The diameter of each of these grooves is substantially equal to the diameter of the insertion portion 21. For example, the curvature radius of the first groove 52a is smaller than the curvature radius of the second groove 52b, and the curvature radius of the second groove 52b is smaller than the curvature radius of the third groove 52c.

The calibrator 50 according to the present modification is disposed so that the bottoms of the first groove 52a, the second groove 52b, and the third groove 52c are down. During calibration, the insertion portion 21 is disposed along the first groove 52a, the second groove 52b, or the third groove 52c. As a result, the shape of the insertion portion 21 is restrained in a known curving shape. The configuration is similar in other respects to that in the first embodiment.

According to the present embodiment, the insertion portion 21 fitted in the calibrator 50 is more easily visually recognized than in the first modification of the first embodiment. Moreover, unnecessary objects such as dust adhering to the groove 52 of the calibrator 50 can be easily removed. Even if the insertion portion 21 is not inserted from the distal side of the insertion portion 21 in order as in the first modification of the first embodiment when the insertion portion 21 is disposed in the calibrator 50, the insertion portion 21 can be fitted into the groove 52 from the side surface of the insertion portion 21 according to the present modification. As a result, the insertion portion 21 can be more easily disposed in the calibrator 50. In other respects, advantageous effects similar to those in the first modification of the first embodiment are also obtained according to the present modification.

Even the calibrator 50 provided with the groove as in the present modification satisfies the following condition. That is, when the insertion portion 21 is fitted in the groove 52, the longitudinal axis of the groove 52 aligns with the longitudinal axis of the insertion portion 21. The direction from the longitudinal axis toward the bottom is defined as one direction of the second axis. The first axis perpendicular to the longitudinal axis and the second axis is defined. The groove 52 restrains the deformation and movement of the fitted insertion portion 21 in both directions along the first axis (e.g., both right and left directions). Further, the groove 52 restrains the deformation and movement of the fitted insertion portion 21 in one direction along the second axis (e.g., downward direction).

It should be understood that as long as the insertion portion 21 does not come off the groove 52 due to gravity, the calibrator 50 may be obliquely disposed even if the bottom of the groove does not face down. The sectional shape of the groove may be an arc having a central angle of 180° or more. In this case, the placement angle of the calibrator 50 may be any angle. Moreover, in this case, when the calibrator 50 is elastic, the insertion portion 21 can be fitted in from the side surface of the insertion portion 21 even if the insertion portion 21 is not inserted from the distal end along the groove.

As in the first modification of the first embodiment, one calibrator 50 is provided with three grooves: the first groove 52a, the second groove 52b, and the third groove 52c. However, the calibrator is not limited to this. As in the first embodiment, calibrators that are each provided with one of the first groove 52a, the second groove 52b, and the third groove 52c may be prepared.

### [Second Embodiment]

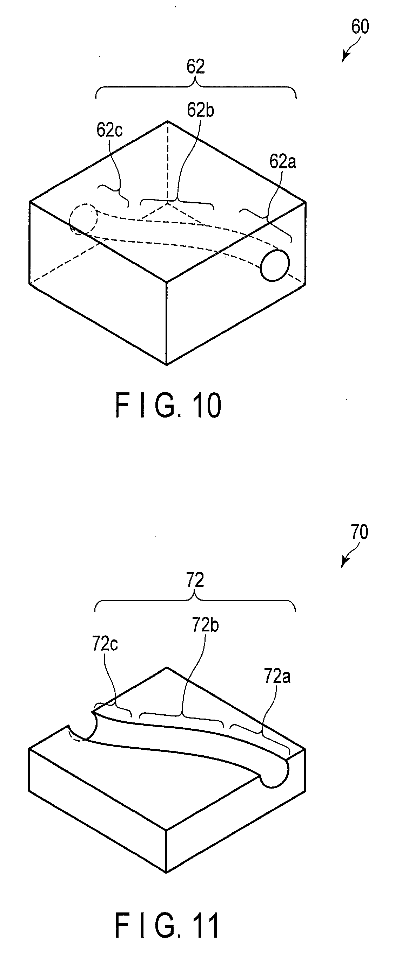

A second embodiment is described. The differences between the second embodiment and the first embodiment are described here, and the same parts are provided with the same reference signs and are not described. As shown in FIG. 10, a calibrator 60 according to the present embodiment comprises a through-hole 62. The through-hole 62 includes a first region 62a, a second region 62b, and a third region 62c that are different in curvature radius. For example, the curvature radius of the first region 62a is Rb, the curvature radius of the second region 62b is infinite, and the curvature radius of the third region 62c is Ra. That is, the calibrator 60 according to the present embodiment has a configuration in which the second calibrator 30b, the third calibrator 30c, and the first calibrator 30a according to the first embodiment are connected in series. When the insertion portion 21 is inserted in the calibrator 60 according to the present embodiment and moves in the longitudinal direction, the insertion portion 21 curves with varying curvature radii in turn. Thus, the first region 62a, the second region 62b, and the third region 62c function as restraining portions configured to restrain the insertion portion 21.

The length of the through-hole 62 according to the present embodiment is shorter than, for example, the space between the respective curve detection units 22 provided in the insertion portion 21. That is, one or less curve detection unit 22 is only disposed in the calibrator 60. In the calibration operation, the curve detection unit 22 moves in the first region 62a, the second region 62b, and the third region 62c in order. Calibration in a situation in which the curvature radius is restrained to Rb is made when the curve detection unit 22 is located in the first region 62a. Calibration in a situation in which the curvature radius is restrained to infinity is made when the curve detection unit 22 is located in the second region 62b. Calibration in a situation in which the curvature radius is restrained to Ra is made when the curve detection unit 22 is located in the third region 62c.

According to the calibrator 60 in the present embodiment, in the calibration operation, calibration in multiple curving states can be made by one calibrator 60 even if multiple calibrators are not used in turn. According to the present embodiment, the calibration operation can be efficiently performed.

In the above description, the length of the through-hole 62 is shorter than the space between the curve detection units 22. However, the length of the through-hole 62 is not limited to this. When the calibration operations for the multiple curve detection units 22 can be simultaneously performed, the length of the through-hole 62 may be equal to or more than the space between the curve detection units 22. For example, the space between the first region 62a and the second region 62b or the space between the second region 62b and the third region 62c has only to correspond to the space between the respective detectors of the curve detection units 22. In this instance, for example, while the first detector 22a is located in the third region 62c, the second detector 22b is located in the second region 62b, and the third detector 22c is located in the first region 62a. As a result, the detectors included in the curve detection unit 22 can be simultaneously calibrated. That is, according to the present embodiment, the calibration operation can be highly efficiently performed.

### [Modification of Second Embodiment]

A modification of the second embodiment is described. The differences between the modification and the second embodiment are described here, and the same parts are provided with the same reference signs and are not described. Although the calibrator 60 is provided with the through-hole 62 which varies in curvature radius from region to region in the second embodiment, a calibrator 70 is provided with a groove 72 which varies in curvature radius from region to region in the present modification as shown in FIG. 11. The relation between the second embodiment and its modification is equivalent to the relation between the first modification and the second modification of the first embodiment. The configuration is similar to that in the second embodiment except that the through-hole is changed to the groove. That is, the groove 72 according to the present modification includes a first region 72a in which the curvature radius is Rb, a second region 72b in which the curvature radius is infinite, and a third region 72c in which the curvature radius is Ra.

Functions and advantageous effects similar to those in the second embodiment can also be obtained in the present modification. Moreover, advantageous effects similar to those in the second modification of the first embodiment are obtained.

### [Third Embodiment]

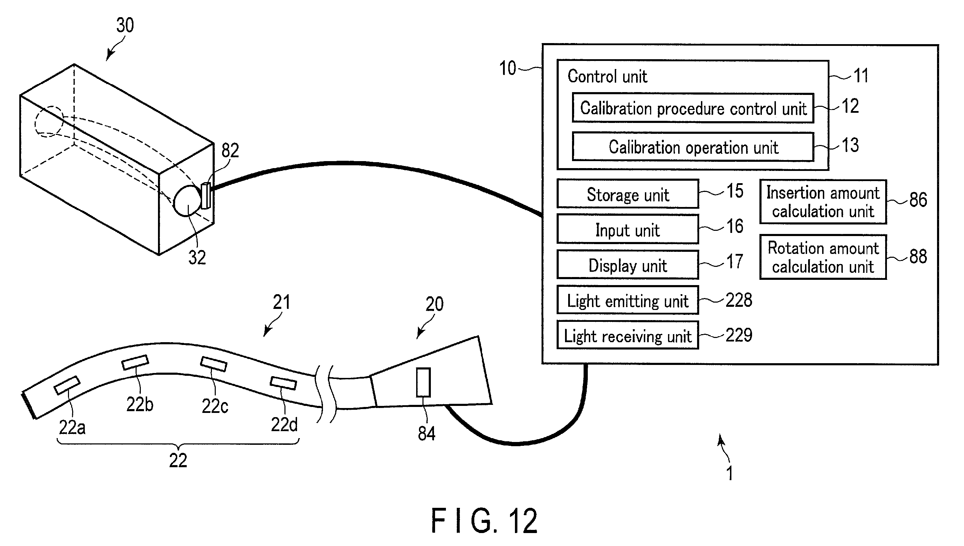

A third embodiment is described. The differences between the third embodiment and the first embodiment are described here, and the same parts are provided with the same reference signs and are not described. An overview of a configuration example of the endoscope system 1 according to the present embodiment is shown in FIG. 12. In the endoscope system 1 according to the present embodiment, a linear encoder 82 as a distance sensor for detecting the insertion amount is provided in the vicinity of the through-hole 32 of the calibrator 30. This linear encoder 82 outputs a signal regarding the amount of the insertion of the insertion portion 21 into the through-hole 32.

The linear encoder 82 is connected to the main unit 10. The main unit 10 is provided with an insertion amount calculation unit 86 as a position calculation unit. The insertion amount calculation unit 86 calculates an insertion amount on the basis of the signal output from the linear encoder 82. The insertion amount calculation unit 86 outputs the insertion amount to the calibration procedure control unit 12. Thus, for example, the linear encoder 82 and the insertion amount calculation unit 86 function as position identifying members to identify the position of the curve detection unit 22 relative to the calibrator 30.

In the endoscope system 1 according to the present embodiment, an acceleration sensor 84 for detecting a rotation amount is provided in the endoscope 20. The acceleration sensor 84 detects an acceleration of gravity and thereby outputs a signal regarding the rotation amount of the insertion portion 21.

The main unit 10 is provided with a rotation amount calculation unit 88. The rotation amount calculation unit 88 calculates a rotation amount on the basis of the signal output from the acceleration sensor 84. The rotation amount calculation unit 88 outputs the rotation amount to the calibration procedure control unit 12. Instead of the above-mentioned acceleration sensor, for example, a rotary encoder provided in the calibrator 30 may be used to acquire the rotation amount. Thus, for example, the acceleration sensor 84 or the rotary encoder and the rotation amount calculation unit 88 function as rotation identifying members to identify the rotation amount of the curve detection unit 22 relative to the calibrator 30.

The calibration procedure control unit 12 controls a calibration procedure on the basis of the insertion amount input from the insertion amount calculation unit 86 and the rotation amount input from the rotation amount calculation unit 88. That is, the insertion amount and the rotation amount are used in, for example, the determination of the positional relation between the calibrator 30 and the insertion portion 21 in step S104 of the processing described with reference to FIG. 5. When a predetermined positional relation is obtained, the user may be informed of this fact. Alternatively, when a predetermined positional relation is obtained, the processing may progress to step S105.

When the positional relation between the calibrator 30 and the insertion portion 21 rapidly changes, calibration may not be properly made. In this case, the processing may not progress to step S105.

According to the present embodiment, even if the user does not manually adjust the positional relation between the insertion portion 21 and the calibrator 30 and does not input an instruction to the main unit 10, a calibration operation is performed on the basis of the outputs of the linear encoder 82 and the acceleration sensor 84. As a result, the time for the user operation is saved, and a simple and easy calibration operation is achieved.

### [Modification of Third Embodiment]

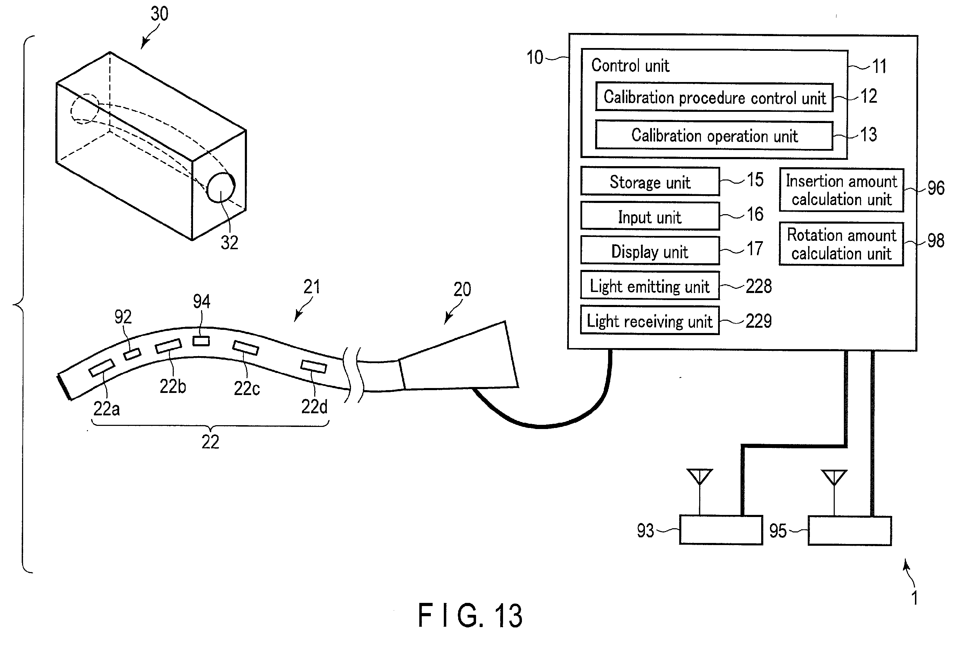

A modification of the third embodiment is described. The differences between the modification and the third embodiment are described here, and the same parts are provided with the same reference signs and are not described. An overview of a configuration example of the endoscope system 1 according to the present modification is shown in FIG. 13. In the third embodiment, the linear encoder 82 and the acceleration sensor 84 are used to detect the insertion amount and the rotation amount. In contrast, according to the present modification, for example, a magnetic direction and position detection system is used. That is, the insertion portion 21 is provided with, for example, a position marker 92 and a direction marker 94. Moreover, the endoscope system 1 is provided with a position detector 93 and a direction detector 95. The position detector 93 and the direction detector 95 are each connected to the main unit 10. The main unit 10 is provided with an insertion amount calculation unit 96 and a rotation amount calculation unit 98.

The position marker 92 and the direction marker 94 include, for example, magnetic coils. The position detector 93 detects a magnetic field generated by the position marker 92, and outputs the detection result to the insertion amount calculation unit 96. The direction detector 95 detects a magnetic field generated by the direction marker 94, and outputs the detection result to the rotation amount calculation unit 98. The insertion amount calculation unit 96 calculates an insertion amount on the basis of the signal acquired by the position detector 93. The rotation amount calculation unit 98 calculates a rotation amount on the basis of the signal acquired by the direction detector 95. The position marker 92 and the direction marker 94, for example, do not exclusively use magnetism. In the present modification, various methods of identifying positions and directions can be used. Thus, for example, the position marker 92 and the position detector 93 function as position identifying members to identify the position of the curve detection unit 22 relative to the calibrator 30. The direction marker 94 and the direction detector 95 function as rotation identifying members to identify the rotation amount of the curve detection unit 22 relative to the calibrator 30.

The configuration is similar in other respects to that in the third embodiment. According to the present modification, advantageous effects similar to those in the third embodiment are obtained.

In the third embodiment and its modification, the combination of an instrument used for the insertion amount detection and an instrument used for the rotation amount detection is not restricted. One of the insertion amount and the rotation amount may be manually adjusted by the user, for example, as in the first embodiment. The calibrator 30 used in the third embodiment and its modification may be the calibrator according to the aspect of any one of the first embodiment, the second embodiment, and their modifications.

## Claims

1. A calibration assist apparatus for use in a curving system, the curving system including an elongated flexible portion and a curve detection unit which is provided in the flexible portion and which is configured to detect a curving amount of the flexible portion, the calibration assist apparatus assisting in calibrating the curve detection unit, the calibration assist apparatus comprising:
a calibrator configured to restrain a deformation and a movement of the flexible portion in both directions along a first axis perpendicular to a longitudinal axis of the flexible portion and restrain a deformation and a movement of the flexible portion in at least one direction along a second axis perpendicular to the longitudinal axis and the first axis.

2. The calibration assist apparatus according to claim 1, further comprising a position identifying member to identify a position of the curve detection unit relative to the calibrator.

3. The calibration assist apparatus according to claim 2, wherein the position identifying member includes an index which indicates the position of the curve detection unit relative to the calibrator.

4. The calibration assist apparatus according to claim 2, wherein the position identifying member includes
a distance sensor which detects a distance from a predetermined reference position of the flexible portion, and
a position calculation unit which calculates the position of the curve detection unit relative to the calibrator based on an output of the distance sensor.

5. The calibration assist apparatus according to claim 2, wherein the position identifying member includes
a position marker buried in the flexible portion,
a position detector which detects a position of the position marker, and
a position calculation unit which calculates the position of the curve detection unit relative to the calibrator based on an output of the position detector.

6. The calibration assist apparatus according to any one of claims 1 to 5, further comprising a rotation identifying member to identify a rotation amount of the curve detection unit relative to the calibrator.

7. The calibration assist apparatus according to claim 6, wherein the rotation identifying member includes an index which indicates the rotation amount of the curve detection unit relative to the calibrator.

8. The calibration assist apparatus according to claim 6, wherein the rotation identifying member includes
a rotation marker buried in the flexible portion,
a rotation detector which detects an angle of the rotation marker, and
a rotation amount calculation unit which calculates the rotation amount of the curve detection unit relative to the calibrator based on an output of the rotation detector.

9. The calibration assist apparatus according to claim 1, wherein the calibrator has a through-hole configured so that the flexible portion is inserted therethrough.

10. The calibration assist apparatus according to claim 9, wherein the calibrator is provided with a transparent portion or a cutout which is provided so that at least part of an inside of the through-hole is visually recognizable.

11. The calibration assist apparatus according to claim 1, wherein the calibrator has a groove configured so that the flexible portion is inserted therethrough.

12. The calibration assist apparatus according to claim 9, further comprising an index which indicates a curvature and/or a curving direction of the through-hole.

13. The calibration assist apparatus according to claim 1, wherein the calibrator includes restraining portions configured to restrain the flexible portion, each of the restraining portions restraining the flexible portion in different shapes.

14. The calibration assist apparatus according to claim 13, wherein the restraining portions are arranged in series.

15. The calibration assist apparatus according to claim 14, wherein
the curve detection unit includes detectors provided along the longitudinal axis of the flexible portion, and
an entire length of the restraining portions arranged in series is shorter than a space between the detectors.

16. The calibration assist apparatus according to claim 14, wherein
the curve detection unit includes detectors provided along the longitudinal axis of the flexible portion, and
an entire length of the restraining portions arranged in series is equal to or more than a space between the detectors.

17. A curving system comprising:
an elongated flexible portion;
a curve detection unit which is provided in the flexible portion and which detects a curving amount of the flexible portion;
a calibrator configured to restrain a deformation and a movement of the flexible portion in both directions along a first axis perpendicular to a longitudinal axis of the flexible portion and restrain a deformation and a movement of the flexible portion in at least one direction along a second axis perpendicular to the longitudinal axis and the first axis; and
a calibration operation unit which calibrates the curve detection unit based on an output of the curve detection unit in a situation in which the flexible portion is restrained by the calibrator and a shape of the flexible portion in the situation in which the flexible portion is restrained.

18. A calibration method comprising:
restraining a deformation and a movement of an elongated flexible portion in both directions along a first axis perpendicular to a longitudinal axis of the flexible portion and restrain a deformation and a movement of the flexible portion in at least one direction along a second axis perpendicular to the longitudinal axis and the first axis using a calibrator;
acquiring an output of a curve detection unit which is provided in the flexible portion and which is configured to detect a curving amount of the flexible portion in a situation in which the flexible portion is restrained by the calibrator; and
calibrating the curve detection unit based on a shape of the flexible portion in the restraint state and the output.
